Europäisches Patentamt

European Patent Office

Office européen des brevets

(11) Publication number: **0 466 954 A1**

# EUROPEAN PATENT APPLICATION

(21) Application number: **90113680.4**

(22) Date of filing: **17.07.90**

(51) Int. Cl.5: **B01D 3/00**, B01J 8/02,
B01J 35/04, C07C 1/20,
C07C 2/28, C07C 7/148,
C07C 11/02, C07C 11/09,
C07C 41/06, C07C 41/42,
C07C 43/04

(43) Date of publication of application:
**22.01.92 Bulletin 92/04**

(84) Designated Contracting States:
**DE FR GB IT NL**

(71) Applicant: **CHEMICAL RESEARCH &
LICENSING COMPANY**
**1603 Galveston Street**
**South Houston, Texas 77587(US)**

(72) Inventor: **Smith, Lawrence A., Jr.**
**1500 Strawberry Street Harris County**
**Pasedena Texas 77502(US)**

(74) Representative: **Jukes, Herbert Lewis**
**Swann, Elt & Company 31 Beaumont Street**
**Oxford OX1 2NP(GB)**

(54) **Catalytic distillation.**

(57) A method of carrying out chemical reactions in a reaction-distillation column, wherein the materials of a reaction mixture are separated by distillation, the method being used with reactants, products and inerts which are liquid or gaseous under the conditions of reaction, wherein a catalyst system is arranged and supported in said reaction-distillation column, said method being characterised in that said catalyst system comprises a plurality of closed cloth pockets (32) containing a particulate catalytic material (33), the pockets being arranged and supported by wire mesh (31) intimately associated with said closed cloth pockets, said plurality of said pockets being contained in a single cloth belt, said cloth belt being coiled into a spiral and having said wire mesh disposed between coils of said cloth belt spiral.

fig.3

## CATALYTIC DISTILLATION

The present invention relates to conducting catalytic chemical reactions wherein separation of materials in the reaction mixture, i.e., product(s), by-product(s) or starting material(s) may be obtained by concurrent distillation or fractionation thereof.

In a specific application the present in vention relates to the separation of isoolefins from streams containing mixtures of an isoolefin and the corresponding normal olefin. The present invention is especially useful for the separation of isobutene from streams containing n-butenes.

One species of the present invention relates to the preparation of methyl tertiary butyl ether from streams containing mixtures of an isobutene and normal $C_4$ olefin. The present invention is especially useful for the separation of isobutene from streams containing n-butenes.

Isoolefins of 4 carbon atoms are difficult to separate from the corresponding normal olefin by simple fractionation because of the closeness of their boiling points. In prior art processes as generally practiced commercially, the isoolefin is selectively absorbed by sulfuric acid and the re sulting isoolefin-containing sulfuric acid extract is then diluted and heated or treated with steam to separate the isoolefin.

Isobutene and diisobutene are of significant value having diverse applications, for example, iso butene is one of the comonomers for butyl rubber and diisobutene is an intermediate in the preparation of detergents. The n-butenes are required in pure form for homopolymerization and as feeds for the oxidative production of butadiene. One manner of separating these components is to pass the mixture through what is called a cold acid extraction procedure wherein the stream is fed into a bath of concentrated sulfuric acid. Separation is achieved by virtue of the solu bility of the isobutene in the sulfuric acid, the n-butenes and other hydrocarbons present passing overhead.

In an improved process reported in U.S. Pat. No. 3,531,539 to Tidwell, the $C_4$ feed stream containing isobutene was contacted with a molecular sieve at an elevated temperature and under sufficient pressure to maintain a liquid phase, wherein the isobutene is converted to diisobutene which is easily separated from the stream by conventional separation techniques.

Methyl tertiary butyl ether (MTBE) has gained a wide acceptance as a non environmentally harmful octane improver for gasolines. One method of separating isobutene or isoolefins in general from mixtures with the corresponding normal olefins and alkanes, has been to etherify the isoolefin with a $C_1$ to $C_6$ primary alcohol in the presence of an acidic cation exchange resin catalyst, separate the low boiling hydrocarbons from the higher boiling ether by fractionation, fre quently followed by decomposition of the ether to re cover the isoolefin. Such procedures are disclosed in U.S. Pat. No.'s 3,121,124; 3,270,081 and 3,170,000.

More recently, in view of the ether octane improving characteristics, similar processes have been disclosed for preparing and recovering the ether, e.g., U.S. Pat. No's 3,726,942 and 3,846,088.

In a variation on these processes disclosed in U.S. Pat. No.'s 3,629,478 and 3,634,534 to Haunschild, the mixture of isoolefin and normal olefin with lower primary alcohols is fed to distillation column in which there are a plurality of zones of acidic ion exchange resin catalyst whereby the isoolefin ether is formed and drops to the bottom of the column while the normal olefins (and paraffins) are distilled overhead. In particular, the catalyst is contained in downcomers where the reaction is to occur and the distillation takes place on the trays of the column.

US4039590 describes a fixed bed reaction of isobutene and methanol in a straight pass liquid phase system. An undesirable minor side reaction is mentioned which forms butenyl ethers. US2043672 discloses the use of an isomerization catalyst to isomerize butene-1 to butene-2 with the isobutene being fractionated. Dimerization is not part of this process.

German patent specification DE-PS 1075613 discloses a distillation column for performing organic chemical reactions in the presence of fine-grain catalysts. The fine-grain catalysts are located in wire baskets, which are arranged in the column in the manner of fillers. The wire baskets may be Raschig rings, whose top and bottom surfaces are closed by screens having a suitable mesh size. Fine-grain fillers may be located on the trays of a column, whose outlets are provided with sloping fine- mesh screens. There is no disclosure of a catalyst system as defined and utilised in the present invention (see below).

Chemiker-Zeitung/Chemische Apparatur, 90 Jg (1966), Nr. 13. pages 443-446 disclose catalytic reactions in ion exchange columns whilst displacing the chemical equilibrium. Those pages review advantages of fine exchange resins used in organic reactions. There is no disclosure of a catalyst system as defined and utilised in the present invention (see below).

The method of the present invention may be used for carrying out chemical reactions wherein the materials of the reaction mixture are separated by distillation. The method may be used with reactants, products and inerts which are liquid or gaseous under the conditions of the reaction. The reactants may be a single material, such as isobutylene which reacts with itself to form diisobutylene in a $C_4$ stream containing normal butenes, wherein the normal butenes are recovered as an overhead and the diisobutylene recovered as bottoms or the reactants may be different materials such as isobutene and methanol described in more detail herein. It is the discovery of the use of the catalyst system as both the catalyst and the distillation packing such that reaction and distillation are occuring concurrently in the fixed catalyst bed, which is the basis of the present invention.

The present invention includes the separation of isoolefins, preferably having four to six carbon atoms, from streams containing mixtures thereof with the corresponding normal olefins. For example, streams containing isobutene and normal butene, isopentene and normal pentene and isohexene and normal hexene.

The present invention provides a method of carrying out chemical reactions in a reaction-distillation column, wherein the materials of a reaction mixture are separated by distillation, the method being used with reactants, products and inerts which are liquid or gaseous under the conditions of reaction, wherein a catalyst system is arranged and supported in said reaction-distillation column, said method being characterised in that said catalyst system comprises a plurality of closed cloth pockets (32) containing a particulate catalytic material (33), the pockets being arranged and supported by wire mesh (31) intimately associated with said closed cloth pockets, said plurality of said pockets being contained in a single cloth belt, said cloth belt being coiled into a spiral and having said wire mesh disposed between coils of said cloth belt spiral."

Said method of the present invention may be characterised in example by being a method of preparing methyl-tertiary-butyl-ether by contacting a mixture containing isobutene and n-butene with methanol in a fixed bed acid cation exchange resin packing thereby catalytically reacting the isobutene with methanol followed by fractionating the resulting mixture, wherein the mixture and methanol are concurrently contacted with the acid cation exchanged resin in a distillation column reactor the acid cation exchange resin beads being placed into a plurality of pockets in a cloth belt in the distillation-reaction zone, followed by concurrently withdrawing the ether obtained from the distillation column reactor at a point below the feed zone of the mixture and methanol and withdrawing the normal butene at a point above the feed zone of the mixture and the methanol.

Said method of the present invention may be characterised in example by being a method of preparing isobutene by contacting methyl-tertiary-butyl-ether with a fixed bed acid cation exchange resin packing thereby catalytically dissociating the methyl-tertiary-butyl-ether into methanol and isobutene, wherein the methyl-tertiary- butyl-ether is contacted with the fixed bed resin packing in a distillation column reactor the acid cation exchange resin beads being placed into a plurality of pockets in a cloth belt in the distillation dissociation zone, followed by continuously withdrawing the isobutene from the distillation column reactor at a point above the feed zone of the methyl-tertiary-butyl-ether and methanol at a point below said feed zone.

Said method of the present invention may be characterised in example by being a method of producing diisobutene by contacting methyl-tertiary-butyl-ether with a fixed bed acid cation exchange resin packing thereby catalytically dissociating the methyl-tertiary-butyl-ether into methanol and diisobutene, wherein the methyl- tertiary-butyl-ether is contacted with the fixed bed cation exchange resin packing, in a distillation column reactor the acid cation exchange resin beads being placed into into a plurality of pockets in a cloth belt is the distillation dissociation zone, followed by continuously withdrawing diisobutene and methanol from the distillation column reactor at a point below the feed zone of the methyl-tertiary-butyl-ether .

Said method of the present invention may be characterised in example by being a method of preparing diisobutene contacting a C4 hydrocarbon stream containing isobutene with a fixed bed acid cation exchange resin packing thereby catalytically reacting isobutene with itself to form diisobutene, wherein the stream is contacted with a fixed bed resin packing in a distillation column reactor the acid cation exchange resin beads being placed into a plurality of pockets in a cloth belt in the distillation zone, followed by continuously withdrawing at a point above the feed zone of the $C_4$ hydrocarbon stream containing isobutene and said diisobutene is withdrawn at a point below said feed zone.

Said method of the present invention may be characterised in example by being a method for separating isoolefins from a mixture containing isoolefins and normal olefin by contacting the mixture with a fixed bed acid cation exchange resin packing thereby catalytically reacting isoolefin with itself to form a dimer, wherein the mixture is contacted with the fixed bed resin packing in a distillation column reactor the acid cation exchange resin beads being placed into a plurality of pockets in a cloth belt in the distillation zone while the resulting mixture of normal olefins and dimer is fractionated, and said dimer is withdrawn from said distillation column reactor at a point below the feed zone of the mixture containing isoolefin and normal olefin.

The catalyst-packing material is a central feature of the invention, clearly without it the process as described can not be carried out. The shaped catalyst which is employed may also be employed in other processes, such as liquid phase contact with aqueous or organic systems to carry out procedures known in the art, and which may be carried out with catalyst in the prior art in other forms such as beads, pellets or the like. It should be appreciated that the distillation functional catalyst compositions of the present invention provide a more open packing as is required for distillation, hence if the present catalyst-packing is employed in a liquid phase process, a larger bed will be required to obtain the comparable degree of liquid-catalyst contact as compared for ex ample to close packed solid beds of small beads.

The particular shape of the catalytic material is not critical, so long as the shape func tions as a distillation packing. Thus, balls, chunks, sheets, tubes or spirals of catalytic material may be employed as well as the conventional distillation shapes noted above.

The catalytic material may be any material appropriate for the reaction at hand, that is, it may be an acid catalyst or a basic catalyst or others such as catalytic metals and their oxides or halides suit able for a multitude of catalytic reactions and of course, the catalyst is heterogeneous with the reactants or other fluids in the system.

For example, a different reaction is the preparation of formic acid from methanol using iron oxide or transesterification using a base catalyst such as NaOAl.

The term "catalyst" or "catalytic material" is used to include any solid material which is recog nized for the reaction under consideration as performing as a catalyst therein.

Thus, the catalytic material is more than that in the present invention. It is a distillation part or component since it is also the distillation packing (note there may be inert distillation packing in the column also, such as, below or above the catalytic packing, between beds of catalytic packing or intermixed into the catalytic packing). Thus, broadly stated, the catalytic material is a component of a dis tillation system functioning as both a catalyst and a distillation packing, i.e., a packing for a distillation column having both a distillation function and a catalytic function.

It has been found that the resin beads in a conventional fixed bed form too compact a mass for the upward flowing vapor and downward flowing liquid. How ever, it has been found that by placing the resin beads into a plurality of pockets in a cloth belt, which is supported in the distillation column reactor by open mesh knitted stainless steel wire by twisting the two together, allows the requisite flows, prevents loss of catalyst, allows for the normal swelling of the beads and prevents the breakage of the beads through mechanical attrition. This novel catalyst arrangement is also part of the present invention.

The cloth may be of any material which is not attacked by the hydrocarbon feed or products under the conditions of the reaction. Cotton or linen may be used, however, fiber glass or "teflon" cloth are pre ferred. Briefly, a preferred catalyst system comprises a plurality of closed cloth pockets containing parti culate catalytic material arranged and supported in said distillation column reactor by wire mesh intimately associated therewith.

The particulate catalytic material may be a powder, small irregular fragments or chunks, small beads and the like. The paticular form of the cata lytic material in the cloth pockets is not critical, so long as sufficient surface area is provided to allow a reasonable reaction rate. This sizing of catalyst particles can be best determined for each catalytic material (since the porosity or available internal surface area will vary for different materials and of course affects the activity of the catalytic material).

What readily distinguishes the present method from prior art is that the prior art has consistently employed a continuous liquid phase system for contacting the isoolefin with the acidic catalyst, whereas the present invention carries out the method in a catalyst packed distillation column which can be appreciated to contain a vapor phase and some liquid phase as in any distillation. The dimerization reaction of iso butene and the fractionation of the resultant n-butene- dimer mixture is carried out simultaneously, i.e., con currently. That is as the dimer is formed in the catalyst bed, the lower boiling n-butene is fractionated away in the catalyst bed and removed overhead while the high boiling dimer drops to the lower portion of the column.

The bulk type liquid phase reactions of the prior art had as one problem the control of the tempera ture. The distillation avoids this problem entirely.

Thus, the catalyst packing is of such a nature as to allow the vapor flow through the bed, yet provide a sufficient surface area for catalytic contact. In the process described in the earlier mentioned Haunschild patents, the catalyst is packed into the down comers and maintained in what is a flooded state as the liquid in the column passes down through the downcomer to the next lower tray. The material from the downcomer is then fractionated on the lower tray as in a conven tional tower.

For example, according to the present invention a higher boiling reactant is continually contacted with the catalyst and lower boiling reactants passing upward in the fixed catalyst bed which provides more opportunity for reaction. Similarly, the removal of a reaction component from the reaction mixture of a reversable reaction, forces the reaction to completion. Thus, in procedures where catalytic reaction and fractionation are features, the present process provides both func tions in a single step. The equipment is very simple, and can utilize any type of distillation tower (pro vided the fixed beds of catalyst can be inserted therein to fill the reaction-distillation zone). It can be appreciated that any packing will restrict the vapor flow in the column and that if alternate unpacked avenues of travel are provided, the vapor will follow the route of least resistance. Thus, in a reactor confirguration such as shown in the Haunschild patents, no vapor contact or flow could or would proceed through the catalyst packed downcomers.

The present process is also useful for reacting one material in a multicomponent feed and concurrently separating unreacted components of the feed therefrom by fractionation.

The fractionation may be directed to recovering a product overhead or as a bottom fraction. It may also be directed to recovering a non reactant such as n-butene from the reaction mixture of isobutene and methanol, the n-butene being a feed stream component which is otherwise difficult to separate from the isobutene. Thus, the present disclosed process may be used for specific reactions between relatively pure reactants or the selective reaction and recovery of desired pro ducts from mixed feeds.

It is to be expected that for any particular feed stream and selective reaction therein, the specific conditions of reaction will need to be determined by some minimum amount of experimentation employing the invention as described herein and using information provided herein to conduct the process in accordance with the present invention. Similarly, although the illustrations are primarily directed to mixed $C_4$ streams wherein isobutene is selectively reacted, other streams and processes are contemplated, as for example, iso merizations of butene-2 to butene-1 and the concurrent fractionation of the resulting isomerization mixture. Furthermore, in addition to dimerization, etherification and isomerization, all other types of reactions are contemplated within the scope of the process, for example, esterification, chlorination, hydration, dehydrohalo genation, alkylation, polymerization, and the like.

The reactants (and inerts) are generally organic compounds (although some inorganic compounds may be dissolved therein). Although $C_4$ hydrocarbons are illustrated, it is contemplated that organic compounds which are fluid and produce reaction products which are fluid under the conditions of operation and which are subjected to fractionation for separation or recovery of materials from the reaction are all suitable for use in the present process.

Thus, in its generic form, the present invention enables concurrently conducting chemical re actions to produce a reaction mixture and fractionation of the reaction mixture comprising:
(a) feeding reactants to a distillation column reactor into a feed zone,
(b) concurrently:
(1) contacting said reactants with a fixed bed catalyst packing in a form which provides dis tillation sites in a distillation-reaction zone, thereby catalytically reacting said reactants to form a reaction mixture,
(2) fractionating the resulting reaction mixture in said fixed bed catalyst to recover a lower boiling fraction of said reaction mixture overhead and a higher boiling fraction thereof as a bottom,
whereby said reaction and fractionation are occurring concurrently within the fixed catalyst bed which serves as both catalyst and distillation packing in said dis tillation column reactor.

Furthermore, the catalytic material must be in a form to serve as a distillation packing, for example rings, saddles, balls, irregular pieces, sheets, tubes, spirals, packed in bags.

Broadly stated, the catalytic material is a component of a distillation system functioning as both a catalyst and distillation packing, i.e., a packing for a distillation column having both a distilla tion function and a catalytic function.

One species of the present invention is a method for the preparation of methyl tertiary butyl ether from streams containing mixtures of isobutene and normal butene.

The use of the invention for producing methyl tertiary ether comprises (a) feeding a mixture containing an isobutene and normal butene to a distilla tion column reactor into a feed zone, (b) feeding methanol into said feed zone (c) concurrently: (1) contacting said mixture and methanol with a fixed bed acidic cation exchange resin packing in a distillation-reaction zone, thereby catalytically reacting the isobutene with methanol to form methyl tertiary butyl ether, and (2) fraction ating the resulting mixture of ether and normal olefin, (d) withdrawing the ether from the distillation column reactor at a point below said feed zone and (e) with drawing the normal olefin from the distillation column reactor at a point above the feed zone, preferably above the acidic cation exchange resin.

A particular embodiment of the present invention is for the separation of isobutene from a mix ture comprising n-butene and isobutene. More generally, the invention is suitable for the separation of iso butene from a hydrocarbon stream which is substantially $C_4$ hydrocarbons, such as n-butane, isobutene, n-butene, isobutane, and butadiene (minor amounts of $C_3$ and $C_5$ hydrocarbons, i.e., less than 10% may be incidental components of such $C_4$ stream).

Briefly stated, separating isobutene comprises:

(a) feeding a mixture containing isobutene and n-butene and methanol to a distillation column reactor into a feed zone,

(b) concurrently:

(1) contacting said mixture and methanol with a fixed bed acidic cation exchange resin packing in a distillation-reaction zone, thereby catalytically reacting isobutene with methanol to form methyl tertiary butyl ether, and

(2) fractionating the resulting mixture comprising methyl tertiary butyl ether and n-butene,

(c) withdrawing said methyl tertiary butyl ether from said distillation column reactor at a point below said feed zone and,

(d) withdrawing n-butene from said distillation column reactor at a point above the feed zone.

The reaction system can be described as heterogeneous since the catalyst remains as a distinct entity. The catalyst may be employed in such conventional distillation packing shapes, as Raschig rings, Pall rings, saddles or the like. Similarly, the resin may be employed in a granular or bead form as described herein.

The methanol may be and is preferably present in a stoichiometric amount although an excess of up to 10%, may be desirable. In addition, slightly less than a stoichiometric amount may be employed. It should be appreciated that the skilled chemist will optimize the proportions and precise conditions for each particular piece of equipment and variation in catalyst, once the basic invention is comprehended.

It has been found that the resin beads in a conventional fixed bed form too compact a mass for the upward flowing vapor and downward flowing liquid. However, it has been found that placing the resin beads into a plurality of pockets in a cloth belt, which is supported in the distillation column reactor by open mesh knitted stainless steel wire by twisting the two together, allows the requisite flows, prevents loss of catalyst, allows for the normal swelling of the beads and prevents the breakage of the beads through mechanical attrition. This novel catalyst arrangement is described herein.

The etherification reaction of isobutene and the fractionation of the resultant n-butene-ether mixture is carried out simultaneously, i.e., concurrently. That is as the ether is formed in the catalyst bed the lower boiling n-butene is fractionated away in the catalyst bed and removed overhead while the high boiling ether drops to the lower portion of the column.

The bulk type liquid phase reactions of the prior art had as one problem the control of the temperature. The distillation avoids this problem entirely.

The Hauschild patents described above, were an improvement in that there was some etherification catalyst in the column but the etherification and distillation were not concurrent. Etherification occurs only in the liquid phase in the downcomers with distillation occurring subsequently on the tray, thus teaching only a contact with the catalyst in the down flow of the reactants severly limited the reaction and prompted the patentee to suggest multiple conventional fixed bed reactions of isobutene and alcohol to form ether prior to introduction to various trays in the distillation column.

Another species of reaction, which is closely related to the MTBE production is the production of high purity isobutene from the dissociation of MTBE. This reaction is obtained by feeding MTBE to the catalyst bed packing in the reaction-distillation column, recovering methanol bottoms and high purity (95% +) iso butene overhead product, using the same acidic resin catalyst. The feed zone is the upper end of the catalyst bed.

In another closely related species of reaction, the reaction of methanol and isobutene is conducted as described herein, generally, but the amount of methanol is less than stoichiometric amount. By raising temperatures (e.g., increase the pressure in the column) and the dimerization of isobutene is favored. A bottoms product of high purity diisobutene (fewer codimers than in the usual type of process to produce diisobutene from $C_4$ streams) is obtained.

The present invention will be further described, by way of example only, with reference to the accompanying drawings, in which :-

Fig.1 is a schematic representation of a catalytic distillation column for use in this invention.

Fig.2 is an elevational view of a catalyst belt packing.

Fig.3 is a cross sectional view of the catalyst belt packing taken along 3-3 of Fig.2.

Mixed $C_4$ streams containing principally iso butane (I-$C_4$), normal butane (n-$C_4$), butene-1 (B-1), isobutene (I-B), trans butene-2 (TB-2) and cis butene-2 (CB-2) (plus some minor impurities including butadiene), can be treated with cold sulfuric acid to remove isobutene and produce a butylene concentrate. The isobutene removed is recovered from the acid as a polymer (mostly dimer). The isobutene dimer (i.e., some other isobutene polymers as well) and butene concentrate are valuable products.

A substitute route for accomplishing this same separation has been discovered. It has been found that a distillation column packed with a properly supported acid catalyst can produce a bottom stream containing isobutene polymer (most dimer) and an overhead stream that is relatively free of isobutene. This is sur prising since the catalyst used would normally produce mostly heavy isobutene polymers and copolymers if the reaction were conducted in a conventional fixed bed.

It has been found that a distillation column packed with a properly supported acid catalyst into which the mixed $C_4$ stream and methanol are fed can produce a bottom stream containing methyl tertiary butyl ether and an overhead stream that is relatively free of iso butene.

The success of the catalytic distillation approach lies in an understanding of the principles associated with distillation. First, because the re action is occurring concurrently with distillation, the initial reaction product, e.g., methyl tertiary butyl ether (or diisobutylene) is removed from the reaction zone nearly as quickly as it is formed. This removal of the ether (or isobutene dimer) minimized decomposition of the ether and chaining to form isobutene polymer (or further chaining to large polymer lengths is in the case of dimerization). Second, because all the $C_4$ com ponents are boiling, the temperature of the reaction is controlled by the boiling point of the $C_4$ mixture at the system pressure. The heat of reaction simply creates more boil up, but no increase in temperature. Third, the reaction has an increased driving force because the reaction products have been removed and can not contribute to a reverse reaction (LeChatelier's Principle).

As a result, a great deal of control over the rate of reaction and distribution of products can be achieved by regulating the system pressure. Also, ad justing the through-put (residence time = liquid hourly space velocity$^{-1}$) gives further control of product distribution and degree of isobutene removal.

The temperature in the reactor is determined by the boiling point of the $C_4$'s at any given pressure, that is, at constant pressure a change in the tempera ture of the system, indicates a change in the composition in the column. Thus, to change the temperature the pressure is changed. By increasing the pressure, the temperature in the system is increased. Generally, pressures in the range of 0 to 400 psig (1 to 28.59 bar) are or may be employed, preferably 30 to 150 psig (13.08 to 11.35 bar). For the $C_4$ stream, the present reaction will be carried out generally at pressures in the range of 10 to 300 psig (1.70 to 21.70 bar), which will generally mean temperatures in the range of 10 to 100 ° C.

The reaction of isobutene with itself is equilibrium limited; however, by carrying out the reaction in a distillation column reactor and fractionating the formed product (diisobutene) downward away from the reaction zone, the equilibrium is constantly disrupted and hence the reaction never comes to equilibrium. This has the advantage of course, of achieving an effective 100% conversion (provided the catalyst bed is of sufficient length such that none of the isobutene escapes therefrom to go overhead with the n-butenes). The adjustment of the size of the catalyst bed is a mere mechanical step to be determined for each reactor and in accordance with the reaction conditions.

The system would normally be considered anhydrous; however, small amounts of water often satu rate the feed stream and represent about 400 to 600 ppm thereof. The process will continue to operate in the same fashion, in the presence of this amount of water; however, the following effects have been observed: (1) all of the rates increase, however, the lower rates increase faster. (Although not mentioned above, those in the art will recognize that there is a reaction of isobutene with butene to produce "codimer". This rate is normally much slower than the diisobutene rate). (2) the amount of codimer increases and (3) tertiary butanol is produced in small amounts.

The feed to the distillation column reactor is made at the lower end of the catalyst bed, preferably into the catalyst to allow immediate contact of the isobutene with the catalyst.

The reaction of isobutene with methanol is equilibtium limited; however, by carrying out the reaction in a distillation column reactor and fractionating the formed product, methyl tertiary butyl ether (MTBE), downward away from the reaction zone, the equilibrium is constantly disrupted and hence the reaction never comes to equilibrium. This has the advantage of course, of achieving an effective 100% conversion, provided the catalyst bed is of sufficient length such that none of the isobutene escapes therefrom to go overhead with the n-butenes (a problem the Hauschild process does not solve). The adjustment of the size of the catalyst bed is a mere mechanical step to be determined for each reactor and in accordance with the reaction conditions.

The MTBE system would normally be considered anyydrous; however, small amounts of water often satu rate the feed stream and represent about 400 to 600 ppm thereof. The process will continue to operate in the same fashion, in the presence of this amount of water. Generally the system will be employed with less than 1 mole % water in the feed. However, the limitation on water is relevant to the MTBE reaction. Quite obviously, where water is a reactant or a principal component of a feed stream according to the generic invention, it may be present in substantially larger amounts as required,

The feed of the distillation column reactor is made at the lower end of the catalyst bed for the MTBE reaction, preferably into the catalyst to allow immediate contact of the isobutene and methanol with the catalyst which is characterized as the feed zone.

A reflux is preferably included in the system. The reflux ratio could vary over the rate of 1 to 20:1. In practice, the higher ratio may be used to compensate for a short catalyst bed such as required for experi mental work. In commercial size units the catalyst bed would be provided so that lower reflux and hence higher unit productivity could be obtained.

The cation resins are those which have been used in the prior art for this reaction. Catalysts suitable for the new MTBE process are cation exchanger, which contain sulfonic acid groups, and which have been obtained by polymerization or copolymerization of aro matic vinyl compounds followed by sulfonation. Examples of aromatic vinyl compounds suitable for preparing polymers or copolymers are: styrene, vinyl toluene, vinyl naphthalene, vinyl ethylbenzene, methyl styrene, vinyl chlorobenzene and vinyl xylene. A large variety of methods may be used for preparing these polymers; for example, polymerization alone or in admixture with other monovinyl compounds, or by crosslinking with poly vinyl compounds; for example, with divinyl benzene, di vinyl toluene, divinylphenylether and others. The polymers may be prepared in the presence or absence of solvents or dispersing agents, and various poly merization initiators may be used, e.g., inorganic or organic peroxides, persulfates, etc.

The sulfonic acid group may be introduced into these vinyl aromatic polymers by various known methods; for example, by sulfating the polymers with concent rated sulfuric acid or chlorosulfonic acid, or by co polymerizing aromatic compounds which contain sulfonic acid groups (see e.g., U.S. Pat. No. 2,366,007). Further, sulfonic acid groups may be introduced into these polymers which already contain sulfonic acid groups; for example, by treatment with fuming sulfuric acid, i.e.,sulfuric acid which contains sulfur trioxide. The treatment with fuming fulfuric acid is preferably carried out at 0 to $150^\circ$ C, and the sulfuric acid should contain sufficient sulfur trioxide after the reaction. The resulting products preferably contain an average of 1.3 to 1.8 sulfonic acid groups per aromatic nucleus. Particularly, suitable polymers which contain sulfonic acid groups are copolymers of aromatic monovinyl com pounds with aromatic polyvinyl compounds, particularly, divinyl compounds, in which the polyvinyl benzene content is preferably 1 to 20% by weight of the copolymer (see, for example, German Patent Specification 908,247).

The ion exchange resin is preferably used in a granular size of about 0.25 to 1 mm, although particles from 0.15 mm up to about 1 mm may be employed. The finer catalysts provide high surface area, but also result in high pressure drops through the reactor. The macroreticular form of these catalysts is preferred because of the much larger surface area exposed and the limited swelling which all of these resins undergo in a non-aqueous hydrocarbon medium.

Similarly, other acid resins are suitable, such as perflurosulfonic acid resins which are copolymers of sulfonyl fluorovinyl ethyl and fluorocarbon and des cribed in greater detail in DuPont "Innovation", Volume 4, No. 3, Spring 1973 or the modified forms thereof as described in U.S. Pat. No.'s 3,784,399; 3,770,567 and 3,849,243.

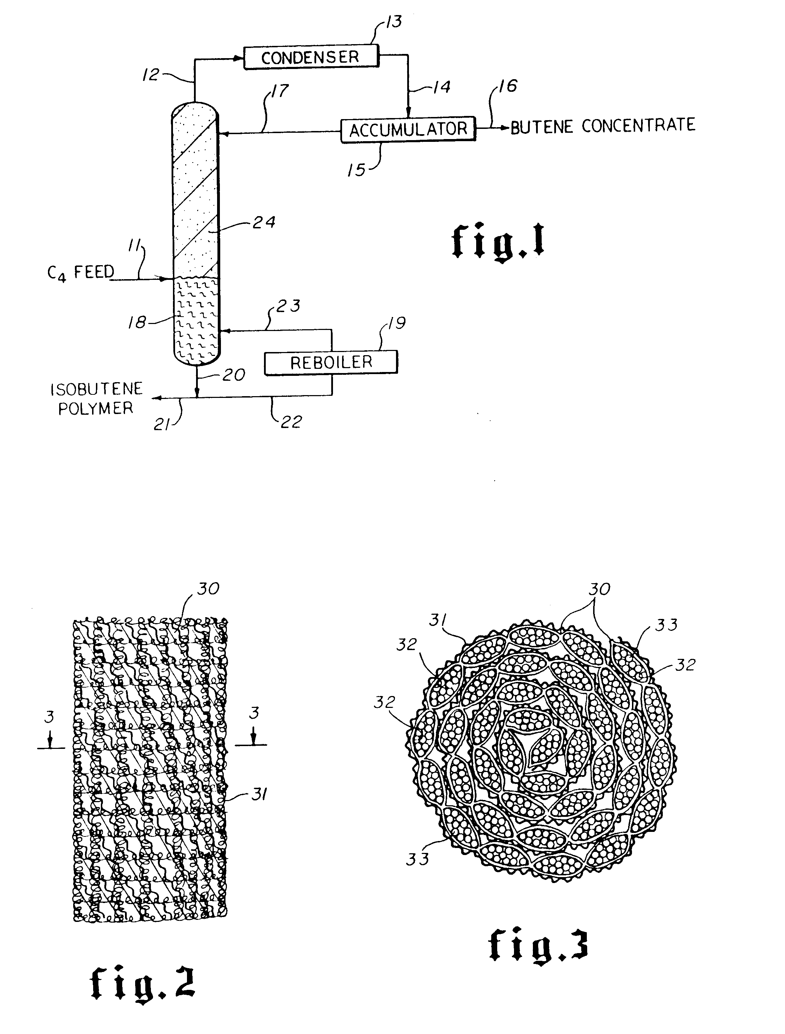

Fig. 1 illustrates schematically a typical distillation column in which the present process may be carried out. The column 10 was a one inch (25.4 mm) diameter, five foot (1.54 m) tall tube containing two feet (0.60 m) of conventional glass 1/6 inch (1.6 mm) helices 18 and three feet (0.91 m) of the catalytic packing as shown in Figs. 2 and 3.

Fig. 2 shows a cloth belt 30 wrapped in open mesh knitted stainless steel wire 31. The cloth bag 30 is composed of a plurality of vertical pockets 32 sewn into the bag. Each pocket 32 is filled with resin catalyst 33.

The wire mesh provides the support for the catalyst (belt) and provides some degree of vapor passage through the catalyst beads, which otherwise form a very compact bed which has a high pressure drop. Thus, the down flowing liquid is in intimate contact with the rising vapors in the column.

In commercial-scale operations, it is con templated, catalyst packing would be made up of alter nating layers of resin-filled cloth belts similar to the ones described above, and a spacing material which could be of any convenient, suitable substance, such as a corrugated wire screen or wire cloth or a knitted wire mesh. The layers would be arranged ver tically to provide vapor passages between the belts. The cylindrical resin-filled pockets could be oriented either vertically or horizontally. For simplicity of fabrication and for better distribution of vapor flow passages, a vertical orientation is preferred. The height of a section of this packing could be of any convenient dimension, from a few inches (mm) to several feet (m). For each of assembly and installation, the packing would be made into sections of the desired shape and size, each section fastened together with circumferential bands or tie wires depending on its size and shape. A complete assembly in a column would consist of several sections, arranged in layers, with the orientation of the resin-filled belts turned at right angles in succes sive layers to improve liquid and vapor flow distribution.

EP 0 466 954 A1

Other configurations which may be useful but with certain draw backs would be cages of wire cloth to contain catalyst beads, immersed in liquid on a conven tional sieve tray. Disadvantages would be the restric tion of vapor flow by the close weave of the wire, which may be compensated by allowing the beads to move freely in the cage, thereby causing attrition. Similarly, suspension of the catalyst on a tray would present problems of attrition, maintaining suspension and pre venting catalyst from leaving the tray.

In the laboratory column the bags are made in the form of a cloth belt approximately six inches (152 mm) wide with narrow pockets approximately ¾ inch (19 mm) wide sewn across the belt. The pockets are spaced about ⅛ inch (3.8 mm) apart. These pockets are filled with the catalyst beads to form approximately cylindrical containers, and the open ends are then sewn closed to confine the beads. This belt is then twisted into a helical form to fit inside the one inch (25.4 mm) column. Twisted in with the belt is also a strip of an open mesh knitted stainless steel wire, which serves to separate the resin filled cloth pockets and provide a passage for vapor flow.

In operation, during the dimerization, the isobutene containing $C_4$ feed enters through line 11 into the lower end of the catalytic zone 24 which con tains the catalyst bag belt 25 as described. The temperature and pressure in the column are such that the $C_4$ boils up in the column, however, as the iso butene contacts the catalyst, dimer is formed, which being higher boiling than the $C_4$ stream, passes to the bottom of the reactor where it is removed via line 20 with a portion being recovered through line 21 and another portion recycled into reboiler 19 through line 22 and hence back into the bottom of the column 10 through line 23.

Meanwhile, the n-butenes pass upward through the catalyst zone 24 and out of the column 10 via line 12 to condenser 13 hence into accumulator 15 via line 14. A portion is recovered as butene concentrate from line 16 and a portion is returned as reflux through line 17 into column 10.

EXAMPLES

In the following examples * , the feed rate of $C_4$'s to the column is adjusted to maintain a bottoms temperature which would correspond to low $C_4$ concentra tion. The catalyst employed was Amberlyst 15, manu factured by Rohm and Haas, Philadelphia Pa. The $C_4$ feed had the following composition:

| Component | mole % |
|---|---|
| Isobutane | 2.8 |
| n-Butane | 8.6 |
| Butene-1 | 24.6 |
| Isobutene | 50.5 |
| Trans-butene-2 | 10.4 |
| Cis-butene-2 | 3.1 |
| Butadiene | .5 |
| ratio butene-1/butene-2 | 1.8 |

The laboratory distillation-reactor column was a one inch (25.4 mm) diameter, five foot (1.54 m) tall tube containing two feet (0.60 m) of conventional glass 1/16 inch (3.2 mm) helices and three feet (0.914 m) of the catalytic packing. The pilot column is 3 inches (76.2 mm) in diameter with 10 feet (3.04 m) of catalyst packing and 5 feet (1.52 m) of conventional ⅜ inch (9.5 mm) pall rings.

In operation, the isobutene containing $C_4$ feed and methanol enters into the lower end of the catalytic zone which contains the catalyst bag belt as described. The temperature and pressure in the column are such that the $C_4$ and methanol boil up in the column, however, as the isobutene and alcohol contact the catalyst, ether is formed, which being higher boiling than the $C_4$ stream, passes to the bottom of the reactor where it is removed. Generally a portion is recovered through and another portion recycled into a reboiler and hence back into the bottom of the column.

Meanwhile, the n-butenes pass upward through the catalyst zone and out of the column to a condenser hence into an accumulator. In normal operation, a portion is recovered as butene concentrate and a por tion is returned as reflux through into the column.

Examples 1, 2, 3, 9 and 10.

9

EXAMPLES 1, 2 and 3

These runs illustrate MTBE production.

| Example | 1 | 2 | 3 |
|---|---|---|---|
| | Laboratory Column | | Pilot Plant Column |
| | LHSV$^{*-1}$ = 1.0 hr | | LHSV$^{-1}$ = 1.4 hr |
| | 115 g (190 ml) cat | | 2640 g (4400 ml) cat |
| System pressure | 80 psig (6.5 bar) | | 100 psig (7.9 bar) |
| Temperature: | | | |
| Bottoms | 228°F (109°C) | | 260°F (127°C) |
| Middle cat. bed | 138°F (59°C) | | 160°F (71°C) |
| Methanol Feed | 2.5 ml/min | | 24 ml/min |
| Reactor Recovery Rate | | | |
| Overhead, grams/hr | 120 | --- | 3060 ml/hr |
| Bottoms, grams/hr | 264 | --- | 4980 ml/hr |
| Analysis, Mole % Overhead | | | |
| Isobutane | 6.1 | 6.6 | 6.1 |
| Normal butane | 18.0 | 15.5 | 15.8 |
| Butene-1 | 40.6 | 40.5 | 47.0 |
| Isobutene | 11.2 | 9.8 | 1.3 |
| Trans-butene-2 | 18.8 | 19.1 | 24.2 |
| Cis-butene-2 | 5.1 | 5.2 | 5.2 |
| Butadiene | .3 | .3 | .3 |
| Bottoms | | | |
| Methanol and $C_4$'s | 5.5 | 7.8 | 1.3 |
| Tertiary Butyl alcohol | 2.5 | .7 | .7 |
| Methyl tertiary butyl ethyl (MTBE) | 93.2 | 91.2 | 91.9 |
| $C_8$'s and heavier | .8 | .3 | 6.1 |
| The process operated according to the present invention gives excellent yields of MTBE at a reasonable feed rate. | | | |

* LHSV$^{-1}$ calculated by dividing overhead take-off rate into the volume of resin in the catalytic zone.

Examples 4 and 5

These examples illustrate the production of high purity isobutene from methyl tertiary butyl ether. The one inch laboratory column was used and packed with 72 grams of catalyst distillation packing as described in pockets of a cloth belt. The catalyst was in the lower portion of the column for this reaction and the feed (MTBE) was at the upper end of the catalyst bed. Two runs were made using two different purities of feed. The conditions and results are set out below:

10

| Example | 4 | 5 |
|---|---|---|
| Feed Mole % MTBE | 95.6% | 99.1% |
| Recovery Rate | | |
| Overhead ml/hr<br>Bottom<br>Pressure, psig | 100<br>not recorded<br>70 (5.84 bar) | 100<br>not recorded<br>80 (6.53 bar) |
| Temperature, $°$ F | | |
| Overhead<br>Bottoms<br>Middle cat. bed | 118 (48$°$C)<br>212 (100$°$C)<br>214 (101$°$C) | 122 (50$°$C)<br>220 (104$°$C)<br>217 (103$°$C) |
| Analysis, %* | | |
| Overhead | | |
| Isobutane<br>Nornal butane<br>Butene-1<br>Isobutene<br>Trans-butene-2<br>Cis-butene-2<br>Butadiene<br>Bottom | .02<br>.4<br>.5<br>96.5<br>1.6<br>.9<br>.03<br>mixture of MTBE and methanol | .05<br>.01<br>.05<br>99.6<br>.2<br>1.2<br>.01<br>mixture of MTBE and methanol |

\* gas phase chromatography area

Examples 6, 7 and 8

Example 6 illustrates the flexibility of the present process to produce high purity diisobutene from the reaction of methanol and isobutene or MTBE only. The three inch (76.2 mm) pilot column was used. In Example 7 the column was modified by adding an additional 5 feet (1.54 m) of column. In Example 6, the column con tained 5 feet (1.54 m) of conventional Pall ring in the lower portion and 10 feet (3.08 m) of the catalyst in cloth bags in the upper portion. In Example 7, an additional 2.5 feet (0.76 m) of catalyst packing was added and 2.5 feet (0.76 m) of $\frac{1}{4}$ inch (6.3 mm) conventional saddles on top of that. In both runs, methanol was fed at the top of the catalyst bed and the $C_4$ feed (as described above) was fed below the catalyst bed. The feed rates were adjusted to obtain the desired product distribution.

In Example 8, high purity methyl tertiary butyl ether (99%) was the feed to column as employed in Example 7. The MTBE was fed into the column at the bottom of the catalyst bed. There was essentially no over head removed except that necessary for analysis. The example demonstrates a unique method for producing diisobutene having a very low codimer contamination. The condition and results of the runs are described below:

| Example | 6** | 7 | 8 |
|---|---|---|---|
| Rate of Recovery | | | |
| Overhead, liters/hr | 4.5 | 3.0 | essentially 0 |
| Bottoms, liters/hr | 4.5 | 4.2 | 4.5 |
| Pressure, psig | 100 (7.91 bar) | 120 (9.29 bar) | 78 (6.39 bar) |
| Temperature °F | | | |
| Overhead | 155 (68°C) | 158 (70°C) | 82 (28°C) |
| Bottoms | 350 (177°C) | 260 (127°C) | 245 (118°C) |
| Middle Cat. bed | 180 (82°C) | 165 (74°C) | 220 (104°C) |
| Analysis %* | | | |
| Overhead | | | |
| Isobutane | 6.5 | 6.2 | - |
| Normal butane | 17.0 | 17.0 | 1.5 |
| Butene-1 | 32.2 | 46.9 | .8 |
| Isobutene | 6.2 | trace | 69.6 |
| Trans-butene-2 | 28.5 | 24.2 | .4 |
| Cis butene-2 | 9.4 | 5.0 | - |
| Butadiene | .2 | .6 | - |
| Dimethyl ether | | | 27.7 |
| Bottoms | | | |
| Lights, methanol } Tertiary butyl alcohol } | .3 | 3.2 1.0 | .6 5.1 |
| MTBE | 32.9 | 95.6 | 68.7 |
| Unknown ether | 2.2 | | .1 |
| Diisobutene | | | |
| 2,4,4-Trimethyl pentene-1 | 40.2 | | 17.6 |
| 2,4,4-Trimethyl pentene-2 | 10.5 | | 5.3 |
| Codimers | 7.1 | | .1 |
| Back flush heavies } Triisobutene } | 7.0 | .2 | 2.4 |

* gas phase chromatography area

**reduced methanol below stoichiometric amount

| Example 9 | | |
|---|---|---|
| System Pressure = 70psig (5.84 bar) | | |
| Catalytic zone temperature = 60°C | | |
| Results: | | |
| OVERHEAD: | | |
| Component | Mole % component at LHSV$^{-1}$ (Hrs)* | |
| | 0.2 | 0.7 |
| isobutane | 5.8 | 4.6 |
| n-butane | 20.0 | 28.7 |
| butene-1 | 31.6 | 15.6 |
| isobutene | 7.4 | 1.5 |
| trans-butene-2 | 26.7 | 37.7 |
| cis-butene-2 | 8.4 | 11.9 |
| butadiene | .18 | .03 |
| ratio butene-1/butene-2 | 1.2 | .3 |
| Overhead take-off (ml/hr) | 480 | 160 |
| bottom temp °C | 133 | 171 |
| bottom $C_4$'s (mole %) | 9.8 | 2.7 |
| BOTTOM:** | | |
| component | | |
| tert-butyl alcohol | .14 | .04 |
| diisobutene | 61.6 | 51.5 |
| codimer | 18.0 | 31.3 |
| triisobutene | 16.8 | 12.9 |
| heavies | 3.5 | 4.2 |

* LHSV$^{-1}$ is calculated by dividing the overhead take-off rate into the volume of resin in the catalytic zone. (The zone contained 115 grams (190 ml) of catalyst).
** $C_4$'s are normalized out

| Example 10 | | |
|---|---|---|
| System Pressure = 40 psig (3.77 bar) | | |
| Catalytic zone temperature = 40°C | | |
| Results: | | |
| OVERHEAD: | | |
| Component | mole % component at LHSV$^{-1}$ (Hrs)* | |
| | 0.7 | 1.4 |
| isobutane | 4.8 | 5.7 |
| n-butane | 15.1 | 16.0 |
| butene-1 | 44.3 | 43.8 |
| isobutene | 12.1 | 9.3 |
| trans-butene-2 | 18.6 | 20.0 |
| cis-butene-2 | 4.8 | 5.1 |
| butadiene | .21 | .2 |
| ratio butene-1/butene-2 | 1.9 | 1.7 |
| Overhead take-off (ml/hr) | 160 | 80 |
| bottom temp °C | 142 | 159 |
| bottom C$_4$'s (mole %) | 3.0 | .4 |
| BOTTOM:** | | |
| Component | | |
| tert-butyl alcohol | .26 | .11 |
| diisobutene | 75.0 | 74.5 |
| codimer | 10.2 | 12.6 |
| triisobutene | 11.6 | 10.2 |
| heavies | 2.9 | 2.6 |

\* LHSV$^{-1}$ is calculated by dividing the overhead take-off rate into the volume of resin in the catalytic zone. (The zone contained 115 grams (190 ml) of catalyst).
\*\* C$_4$'s are normalized out.

## Claims

1. A method of carrying out chemical reactions in a reaction-distillation column, wherein the materials of a reaction mixture are separated by distillation, the method being used with reactants, products and inerts which are liquid or gaseous under the conditions of reaction, wherein a catalyst system is arranged and supported in said reaction-distillation column, said method being characterised in that said catalyst system comprises a plurality of closed cloth pockets (32) containing a particulate catalytic material (33), the pockets being arranged and supported by wire mesh (31) intimately associated with said closed cloth pockets, said plurality of said pockets being contained in a single cloth belt, said cloth belt being coiled into a spiral and having said wire mesh disposed between coils of said cloth belt spiral.

2. A method as claimed in claim 1, characterised by being a method of preparing methyl-tertiary-butyl-ether by contacting a mixture containing isobutene and n-butene with methanol in a fixed bed acid cation exchange resin packing thereby catalytically reacting the isobutene with methanol followed by fractionating the resulting mixture, wherein the mixture and methanol are concurrently contacted with the acid cation exchange resin in a distillation column reactor the acid cation exchange resin beads being placed into a plurality of pockets in a cloth belt in the distillation-reaction zone, followed by concurrently withdrawing the ether obtained from the distillation column reactor at a point below the feed zone of the mixture and the methanol and withdrawing the normal butene at a point above the feed zone of the mixture and the methanol.

3. A method as claimed in claim 1, characterised by being a method of preparing isobutene by contacting methyl- tertiary-butyl-ether with a fixed bed acid cation exchange resin packing thereby catalytically dissociating the methyl-tertiary-butyl-ether into methanol and isobutene, wherein the methyl-tertiary-butyl-ether is contacted with the fixed bed resin packing in a distillation column reactor the acid cation exchange resin beads being placed into a plurality of pockets in a cloth belt in the distillation dissociation zone, followed by continuously withdrawing the isobutene from the distillation column reactor at a point above the feed zone of the methyl-tertiary-butyl-ether and methanol at a point below said feed zone.

4. A method as claimed in claim 1, characterised by being a method of producing diisobutene by contacting methyl- tertiary-butyl-ether with a fixed bed acid cation exchange resin packing thereby catalytically dissociating the methyl-tertiary-butyl-ether into methanol and diisobutene, wherein the methyl-tertiary-butyl-ether is contacted with the fixed bed cation exchange resin packing, in a distillation column reactor the acid cation exchange resin beads being placed into a plurality of pockets in a cloth belt is the distillation dissociation zone, followed by continuously withdrawing diisobutene and methanol from the distillation column reactor at a point below the feed zone of the methyl-tertiary-butyl-ether .

5. A method as claimed in claim 1, characterised by being a method of preparing diisobutene contacting a $C_4$ hydrocarbon stream containing isobutene with a fixed bed acid cation exchange resin packing thereby catalytically reacting isobutene with itself to form diisobutene, wherein the stream is contacted with a fixed bed resin packing in a distillation column reactor the acid cation exchange resin beads being placed into a plurality of pockets in a cloth belt in the distillation zone, followed by continuously withdrawing at a point above the feed zone of the $C_4$ hydrocarbon stream containing isobutene and said diisobutene is withdrawn at a point below said feed zone.

6. A method as claimed in claim 1, characterised by being a method for separating isoolefins from a mixture containing isoolefins and normal olefin by contacting the mixture with a fixed bed acid cation exchange resin packing thereby catalytically reacting isoolefin with itself to form a dimer, wherein the mixture is contacted with the fixed bed resin packing in a distillation column reactor the acid cation exchange resin beads being placed into a plurality of pockets in a cloth belt in the distillation zone while the resulting mixture of normal olefins and dimer is fractionated, and said dimer is withdrawn from said distillation column reactor at a point below the feed zone of the mixture containing isoolefin and normal olefin.

fig.1

fig.2

fig.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 008 860 (CHEMICAL RESEARCH AND LICENSING)<br>* Pages 21-31; claims; figures *<br>– – – | 1-6 | B 01 D 3/00<br>B 01 J 8/02<br>B 01 J 35/04<br>C 07 C 1/20 |
| X | US-A-4 510 336 (D. HEARN)<br>* Complete document *<br>– – – | 1 | C 07 C 2/28<br>C 07 C 7/148<br>C 07 C 11/02 |
| X | US-A-4 504 687 (E.M. JONES)<br>* Claims; column 5, line 58 - column 6, line 28 *<br>– – – – – | 1,2 | C 07 C 11/09<br>C 07 C 41/06<br>C 07 C 41/42<br>C 07 C 43/04 |

TECHNICAL FIELDS
SEARCHED (Int. Cl.5)

B 01 D 3/00
B 01 J 35/00

The present search report has been drawn up for all claims

| Place of search | Date of completion of search | Examiner |
|---|---|---|
| The Hague | 24 April 91 | WRIGHT M.W. |

CATEGORY OF CITED DOCUMENTS

X : particularly relevant if taken alone
Y : particularly relevant if combined with another
    document of the same catagory
A : technological background
O : non-written disclosure
P : intermediate document
T : theory or principle underlying the invention

E : earlier patent document, but published on, or after
    the filing date
D : document cited in the application
L : document cited for other reasons

&: member of the same patent family, corresponding
    document